(19) Europäisches Patentamt

European Patent Office

Office européen des brevets

(11) EP 0 756 867 B1

(12) **FASCICULE DE BREVET EUROPEEN**

(45) Date de publication et mention
de la délivrance du brevet:
**22.10.1997 Bulletin 1997/43**

(51) Int. Cl.$^6$: **A61K 7/48**, A61K 7/06,
A61K 7/04, A61K 7/043

(21) Numéro de dépôt: **96401601.8**

(22) Date de dépôt: **18.07.1996**

(54) **Utilisation d'acides carboxyliques hydroxylés pour le traitement des ongles**

Verwendung von hydroxylierte Carbonsäure zur Behandlung von Nägeln

Use of hydroxylated carboxylic acids for the treatment of nails

(84) Etats contractants désignés:
**AT BE CH DE ES FR GB IT LI NL SE**

(30) Priorité: **31.07.1995 FR 9509306**

(43) Date de publication de la demande:
**05.02.1997 Bulletin 1997/06**

(73) Titulaire: **L'OREAL**
**75008 Paris (FR)**

(72) Inventeurs:
• **Garson, Jean-Claude**
**92150 Suresnes (FR)**
• **Ramin, Roland**
**91760 Itteville (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 442 708**          **EP-A- 0 508 324**
**US-A- 4 588 590**

• RIECHSTOFFE, AROMEN,
KÖRPERPFLEGEMITTEL, vol. 22, no. 11,
Novembre 1972, page 401 XP002003156
"Fingernagelhaut-Ablöser oder Anweicher"

## Description

La présente invention a pour objet l'utilisation d'acides carboxyliques hydroxylés, dans des compositions cosmétiques, pour le traitement des ongles.

Il est bien connu que les ongles présentent, de façon fréquente, des défauts de structure et de consistance, ceux-ci pouvant être d'origines diverses et notamment liés au fonctionnement interne de l'individu, à ses conditions de vie, à son mode d'alimentation, à son âge, à ses états de fatigue ou de surmenage.

Ces défauts peuvent également apparaître sous l'effet d'actions qui érodent, à la suite par exemple d'expositions prolongées ou répétées à des agents détergents, à des solvants, à des produits chimiques en particulier ménager, à des atmosphères chaudes ou froides, humides ou sèches, ou à des expositions aux rayonnements UV.

Ces défauts de structure et de consistance ont pour effet de rendre la surface des ongles inesthétique, ce qui peut être source de gêne et de désagréments multiples.

Les ongles ont alors tendance à durcir, à être secs et cassants, à se dédoubler : ils sont donc fragilisés.

Parmi les traitements des ongles, il est connu selon EP-A-508 324 d'appliquer sur l'ongle une composition comprenant un acide 2-hydroxy carboxylique à une concentration allant de 8 % à 20 % en poids. Ce traitement augmente l'élasticité et la flexibilité de l'ongle. Toutefois, l'utilisation d'acide 2-hydroxy carboxylique à de telles concentration ne permet pas d'obtenir un vernis à ongles présentant de bonnes propriétés cosmétiques ; le film de vernis est long à sécher et reste collant, le film sec est translucide, blanchâtre et peu brillant ; il devient pelliculable et présente donc une mauvaise tenue.

La présente invention a pour but de proposer une composition qui, lorsqu'elle est appliquée sur des ongles, peut permettre d'améliorer leur état général, notamment en en diminuant la dureté, tout en présentant de bonnes propriétés cosmétiques.

La présente invention a donc pour objet l'utilisation, dans une composition cosmétique, d'au moins un acide carboxylique hydroxylé, et/ou d'un de ses sels, comme agent de traitement des ongles, la concentration dudit acide allant de 0,5 % à 3 % en poids par rapport au poids total de la composition.

On a donc constaté que l'utilisation d'acide carboxylique hydroxylé à une concentration allant de 0,5 % à 3 % permet l'obtention d'un meilleur état général des ongles dû notamment au fait qu'ils sont moins fragilisés, car moins secs et/ou cassants, tout en préservant les propriétés cosmétiques de la composition. La composition utilisée selon l'invention s'étale facilement sur l'ongle, sèche rapidement.

Après l'application de la composition sur l'ongle, le film obtenu présente une bonne brillance et une bonne tenue.

Les acides carboxyliques hydroxylés utilisés selon l'invention sont de préférence des acides carboxyliques $\alpha$-hydroxylés.

Ils sont de préférence choisis parmi les acides carboxyliques ayant de 2 à 8 atomes de carbone, en particulier ceux ayant de 2 à 5 atomes de carbone.

Les acides carboxyliques hydroxylés selon l'invention peuvent être linéaires ou ramifiés.

On peut notamment citer les acides glycolique, lactique, malique, tartrique, citrique et/ou mandélique.

On peut utiliser, de préférence, seuls ou en mélange, les acides glycolique, lactique, citrique et mandélique.

Les acides carboxyliques utilisés selon l'invention peuvent être des produits de synthèse ou d'origine naturelle.

Les acides carboxyliques hydroxylés utilisés selon l'invention peuvent se présenter dans la composition finale sous forme d'acide libre et/ou sous la forme d'un de ses sels associés (sels avec une base organique ou un alcalin notamment).

Selon l'invention, la teneur en acides carboxyliques hydroxylés dans la composition utilisée peut aller de préférence de 0,8 % à 2,5 % en poids par rapport au poids total de la composition.

Par ailleurs, la composition cosmétique comprend un support cosmétiquement acceptable.

Ce support peut comprendre des solvants organiques, de l'eau, et/ou un milieu huileux, par exemple.

Comme solvants organiques, on peut citer les cétones, comme l'acétone, la méthyl-éthyl-cétone, la méthyl-isobutyl-cétone; les éthers de glycols; les alcools comme l'éthanol, le n-butanol, le n-propanol, l'isopropanol; les acétates comme l'acétate de butyle, d'éthyle, d'isopropyle, l'acétate de méthoxy-2-éthyle; les hydrocarbures linéaires ou ramifiés tels que l'hexane ou l'octane; ou encore les hydrocarbures aromatiques tels que le xylène et le toluène.

Lorsque le support cosmétiquement acceptable comprend de l'eau, la composition peut se présenter notamment sous la forme d'une solution aqueuse ou hydroalcoolique, d'une émulsion huile-dans-eau ou eau-dans-huile, voire d'une émulsion multiple, ou encore sous la forme d'un gel aqueux.

Le milieu huileux peut comprendre une ou plusieurs huiles, volatiles et/ou non volatiles, par exemple d'origine végétale, minérale, animale et/ou de synthèse, parmi lesquelles on peut citer :

. les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat, les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin ;

. les huiles essentielles naturelles ou synthétiques telles que, par exemple, les huiles d'eucalyptus, de lavandin, de lavande, de vétiver, de citron, de santal, de romarin, de camomille, de sarriette, de noix de muscade, de cannelle, d'hysope, de carvi, d'orange, de géraniol, de cade et de bergamote;

. les hydrocarbures, tels que l'hexadécane et l'huile de paraffine ;

. les esters d'acide minéral et d'un alcool ;

. les éthers et les polyéthers;

. les huiles et gommes de silicones.

De plus, selon l'application envisagée, la composition peut comprendre un polymère filmogène.

Dans le cas d'une application en composition à appliquer sur l'ongle, le polymère filmogène peut permettre de déposer sur l'ongle un film résistant qui assure un contact prolongé de l'acide carboxylique avec la surface de l'ongle.

A titre d'exemple, le polymère peut être choisi parmi la nitrocellulose, l'acétobuty-rate de cellulose, les butyralpolyvinyliques, les résines alkydes, les polyesters, les acryliques et/ou les polyuréthannes.

Les polymères peuvent être dissous ou dispersés dans la composition. Ils peuvent être généralement présents à une teneur allant de 1 % à 40 % en poids par rapport au poids total de la composition.

La composition peut également comprendre, en plus du polymère filmogène, des plastifiants qui permettent de régler la flexibilité du film sans affaiblir sa résistance physique.

Les plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycé-ryle; les citrates de triéthyle, de tributyle, l'acétyl-citrate de tributyle; les phosphates de tributyle, de triphényle; les glycols; le camphre ainsi que leurs dérivés et leurs mélanges. Les plastifiants peuvent être généralement présents à une teneur allant de 1 % à 40 % en poids par rapport au poids total de la composition.

Par ailleurs, la composition peut contenir des adjuvants couramment utilisés dans les compositions cosmétiques. On peut citer à titre d'exemple d'adjuvants, les colorants, les pigments, les nacres, les laques, les agents anti-UV, les agents épaississants, les tensioactifs, les cires, les parfums, et des actifs tels que le D-panthénol, le phytantriol, les vitamines et leurs dérivés, la kératine et ses dérivés, la mélanine, le collagène, la cystine, le chitosane et ses dérivés, les céramides, la biotine, les oligo-éléments, la glycérine, les hydrolysats de protéines, les phospholipides, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition peut se présenter sous forme de composition de maquillage à appliquer sur l'ongle telle qu'un vernis à ongles ou une composition de soin des ongles

On va maintenant donner des exemples illustrant la présente invention.

**Exemple 1:**

On a déterminé la dureté d'un ongle traité par l'acide citrique.

Principe:

On applique sur l'ongle un pénétromètre en forme de pyramide à base carrée, à l'aide d'une charge P. On détermine ensuite la dimension moyenne d'une diagonale de l'empreinte carrée obtenue avec le pénétromètre.

La dureté VICKERS (HV) est alors déterminée par la relation :

$$HV = \frac{1854{,}4 \times P}{d^2}$$

d = diagonale moyenne en $\mu$m
P = charge appliquée en g

La mesure de la dureté VICKERS est effectuée à l'aide du microduromètre M 400 g 2 de la société LECO.

Protocole :

On immerge des fragments d'ongles pendant 3 heures dans de l'eau distillée, puis on laisse à l'humidité ambiante pendant 24 heures.

On applique ensuite 2 µl de solution aqueuse d'acide citrique à 2,5 % en poids sur l'ongle. Les ongles sont ensuite placés 3 jours dans une atmosphère d'humidité relative à 75%. On effectue alors la mesure de la dureté VICKERS.

On effectue une deuxième application du produit et une deuxième mesure de dureté selon les mêmes conditions précédemment décrites.

La solution à 2,5 % en acide citriquea été testée selon ce protocole sur trois échantillons, comparativement à de l'eau (placebo).

Résultats :

On a obtenu les résultats suivants :

| Traitement | Dureté initiale sans traitement | Dureté après la première application | Dureté après la deuxième application |
|---|---|---|---|
| Eau | 11,8 ± 0,5 | 11,2 ± 0,6 | 10,4 ± 0,9 |
| Acide citrique | 12,3 ± 0,3 | 10,4 ± 0,6 (- 15,4 %) | 10,3 ± 0,8 (- 16,3%) |

Les valeurs rapportées correspondent à la moyenne obtenue pour trois échantillons. Les chiffres indiqués entre parenthèse indiquent la diminution de la dureté de l'ongle traité par rapport à l'ongle avant traitement.

On constate que la dureté des ongles, après traitement par l'acide citrique, diminue sensiblement.

Ces essais confirment que les acides carboxyliques hydroxylés ont la propriété de diminuer la dureté des ongles, donc de les ramollir.

**Exemple 2** :

On a comparé trois compositions (A, B, C) comprenant de l'acide citrique à des teneurs différentes (1%, 3%, 8%) par rapport à une même composition (D) exempte d'acide citrique.

Les compositions testées étaient les suivantes :

| Composition | A (invention) | B (invention) | C (hors invention) | D (placébo) |
|---|---|---|---|---|
| Acide citrique | 1 | 3 | 8 | 0 |
| Nitrocellulose | 11 | 11 | 11 | 11 |
| Résines et plastifiants | 16,6 | 16,6 | 16,6 | 16,6 |
| Filtre U. V. | 0,2 | 0,2 | 0,2 | 0,2 |
| Solvants | 71,2 | 69,2 | 64,2 | 72,2 |

On a appliqué chaque composition sur les ongles et on a déterminé la facilité d'application de la composition, le temps de séchage, l'aspect du film après séchage, l'état du film de vernis.

On a obtenu les résultats suivants :

| Composition | A (invention) | B (invention) | C (hors invention) | D (placébo) |
|---|---|---|---|---|
| Application | facile | facile | moins facile | facile |
| Séchage | 15 min | 15 min | 15min | collant après 1 h |
| Aspect du film | transparent | transparent | transparent | translucide |
| Etat du film | bonne tenue | bonne tenue | bonne tenue | film très mou et pelliculable |

On a également déterminé pour chaque composition la brillance, le temps de séchage et la dureté du film de vernis.

La brillance est mesurée pour un film de 150 µm d'épaisseur après séchage sur une plaque thermostatée à 30 °C, à l'aide d'un brillancemètre BYK GARDNER Multiangle, l'angle du faisceau étant de 60 °. Plus la valeur mesurée est élevée, plus le film est brillant.

La dureté est mesurée au pendule de Persoz selon la norme UF-T-30-016.

Le temps de séchage est déterminé en appliquant 150 µm de composition sur un banc thermostaté à 30 °C.

On a obtenu les résultats suivants :

| Composition | A (invention) | B (invention) | C (hors invention) | D (placébo) |
|---|---|---|---|---|
| Brillance | 90 | 70 | 35 | 90 |
| Temps de séchage (min) | 10 à 12 | 10 à 12 | >25 | 4 à 6 |
| Dureté | 50 | 45 | 35 | 100 |

Les résultats obtenus montrent que seules les compositions A et B selon l'invention, comparées à la composition C de l'art antérieur, s'appliquement facilement sur l'ongle et permettent d'obtenir un film de vernis séchant rapidement, transparent et brillant. Le vernis présente également une bonne tenue et n'est pas pelable.

**Exemple 3 :**

On a préparé une base incolore ayant la composition suivante :

| | |
|---|---|
| - nitrocellulose | 15 g |
| - plastifiant et résine | 15 g |
| - alcool isopropylique | 9 g |
| - acide citrique | 1 g |
| - solvant (acétate d'éthyle, acétate de butyle)  qsp | 100 g |

Après application de la composition sur l'ongle et après séchage, on obtient un film lisse et homogène.

On applique cette composition pendant 8 semaines sur des ongles durs, tous les 3 jours.

Avant chaque application, l'ancien film est retiré des ongles à l'aide d'un dissolvant classique.

On constate que les ongles ainsi traités sont assouplis.

**Revendications**

1. Utilisation, dans une composition cosmétique, d'au moins un acide carboxylique hydroxylé, et/ou d'un de ses sels, comme agent de traitement pour diminuer la dureté et/ou ramollir les ongles, caractérisée par le fait que la teneur en acide carboxylique hydroxylé va de 0,5 % à 3 % en poids par rapport au poids total de la composition.

2. Utilisation selon la revendication 1, caractérisée par le fait que l'acide carboxylique hydroxylé est un acide carboxylique $\alpha$-hydroxylé, ou un de ses sels.

3. Utilisation selon l'une des revendications précédentes, caractérisée par le fait que l'acide carboxylique hydroxylé, ou son sel, a de 2 à 8 atomes de carbone.

4. Utilisation selon l'une quelconque des revendications précédentes, caractérisée par le fait que l'acide carboxylique hydroxylé est sous forme acide libre.

5. Utilisation selon l'une quelconque des revendications précédentes, caractérisée par le fait que l'acide carboxylique hydroxylé est choisi parmi les acides glycolique, lactique, malique, tartrique, citrique et/ou mandélique.

6. Utilisation selon l'une quelconque des revendications précédentes, caractérisée par le fait que la composition comprend un support cosmétiquement acceptable choisi parmi les solvants organiques, l'eau et/ou les milieux huileux.

7. Utilisation selon l'une quelconque des revendications précédentes, caractérisée par le fait que la composition comprend un polymère filmogène choisi parmi la nitrocellulose, l'acétobutyrate de cellulose, les butyralpolyvinyliques, les résines alkydes, les polyesters, les acryliques et/ou les polyuréthannes.

8. Utilisation selon la revendication 7, caractérisée par le fait que la composition comprend en outre un plastifiant.

9. Utilisation selon l'une quelconque des revendications précédentes, caractérisée par le fait que la composition se présente sous forme de composition à appliquer sur l'ongle telle qu'un vernis à ongles ou une composition de soin des ongles.

## Claims

1. Use in a cosmetic composition of at least one hydroxy carboxylic acid and/or one of its salts, as a treatment agent for reducing the hardness of and/or softening the nails, characterized in that the content of hydroxy carboxylic acid ranges from 0.5% to 3% by weight relative to the total weight of the composition.

2. Use according to Claim 1, characterized in that the hydroxy carboxylic acid is an $\alpha$-hydroxy carboxylic acid or one of its salts.

3. Use according to one of the preceding claims, characterized in that the hydroxy carboxylic acid or its salt has 2 to 8 carbon atoms.

4. Use according to any one of the preceding claims, characterized in that the hydroxy carboxylic acid is in the form of the free acid.

5. Use according to any one of the preceding claims, characterized in that the hydroxy carboxylic acid is chosen from glycolic, lactic, malic, tartaric, citric and/or mandelic acids.

6. Use according to any one of the preceding claims, characterized in that the composition comprises a cosmetically acceptable vehicle which is chosen from organic solvents, water and/or oily media.

7. Use according to any one of the preceding claims, characterized in that the composition comprises a film-forming polymer chosen from nitrocellulose, cellulose acetobutyrate, polyvinylbutyrals, alkyd resins, polyesters, acrylics and/or polyurethanes.

8. Use according to Claim 7, characterized in that the composition additionally comprises a plasticizer.

9. Use according to any one of the preceding claims, characterized in that the composition is present in the form of a composition to be applied to the nail, such as a nail varnish or nail care composition.

## Patentansprüche

1. Verwendung mindestens einer hydroxylierten Carbonsäure und/oder eines ihrer Salze in einer kosmetischen Zusammensetzung als Mittel zur Behandlung mit dem Ziel, die Härte von Nägeln zu verringern und/oder sie zu erweichen, dadurch gekennzeichnet, daß der Gehalt an hydroxylierter Carbonsäure 0,5 bis 3 Gew.-%, bezogen auf das Gesamtgewicht der Zusammensetzung, beträgt.

2. Verwendung nach Anspruch 1, dadurch gekennzeichnet, daß es sich bei der hydroxylierten Carbonsäure um eine $\alpha$-Hydroxycarbonsäure oder um eines ihrer Salze handelt.

3. Verwendung nach den vorstehenden Ansprüchen, dadurch gekennzeichnet, daß die hydroxylierte Carbonsäure oder ihr Salz 2 bis 8 Kohlenstoffatome aufweist.

4. Verwendung nach einem der vorstehenden Ansprüche, dadurch gekennzeichnet, daß die hydroxylierte Carbonsäure in Form der freien Säure vorliegt.

5. Verwendung nach einem der vorstehenden Ansprüche, dadurch gekennzeichnet, daß die hydroxylierte Carbonsäure unter Glycolsäure, Milchsäure, Äpfelsäure, Weinsäure, Citronensäure und/oder Mandelsäure ausgewählt ist.

6. Verwendung nach einem der vorstehenden Ansprüche, dadurch gekennzeichnet, daß die Zusammensetzung einen kosmetischen Träger umfaßt, der unter organischen Lösungsmitteln, Wasser und/oder öligen Medien ausgewählt ist.

7. Verwendung nach einem der vorstehenden Ansprüche, dadurch gekennzeichnet, daß die Zusammensetzung ein filmbildendes Polymeres enthält, das unter Nitrocellulose, Celluloseacetobutyrat, Polyvinylbutyralen, Alkydharzen, Polyestern, Acrylharzen und/oder Polyurethanen ausgewählt ist.

8. Verwendung nach Anspruch 7, dadurch gekennzeichnet, daß die Zusammensetzung ferner einen Weichmacher enthält.

9. Verwendung nach einem der vorstehenden Ansprüche, dadurch gekennzeichnet, daß die Zusammensetzung in Form einer Zusammensetzung zum Aufbringen auf den Nagel, z.B. als Nagellack oder als Zusammensetzung zur Nagelpflege, vorliegt.